# EUROPÄISCHE PATENTSCHRIFT

(11) **EP 0 716 071 B1**
(45) Veröffentlichungstag und Bekanntmachung des Hinweises auf die Patenterteilung: **06.02.2002**
(21) Anmeldenummer: 95118613.9
(22) Anmeldetag: 27.11.1995
(51) Int. Cl.: C07C 45/59, C07C 49/24, C07C 403/16, A23L 1/275, A23K 1/16

(54) **Herstellung von Beta-Keto-alkoholen**
Preparation of beta-keto-alcohols
Préparation d'alcools bèta-cétoniques

(30) Priorität: 08.12.1994 CH 371394; 18.10.1995 CH 295295
(43) Veröffentlichungstag der Anmeldung: 12.06.1996
(73) Patentinhaber: F. Hoffmann-La Roche AG, 4002 Basel (CH)
(72) Erfinder: Brüngger, Andreas, CH-8604 Volketswil (CH); Gründler, Hansjörg, CH-4310 Rheinfelden (CH); Simon, Werner, CH-4125 Riehen (CH)
(74) Vertreter: Kellenberger, Marcus, Dr.

(56) Entgegenhaltungen:
- EP-A- 0 630 578
- CHEMISTRY LETTERS, 1991, TOKYO JP, Seiten 1377-1378, XP002002863 Y. INOUE ET AL.: "Alpha-Methylene Cyclic Carbonate as a Conjunctive Agent for Aromatic Aldehydes"
- CHEMICAL ABSTRACTS, vol. 067, no. 21, 20.November 1967 Columbus, Ohio, US; abstract no. 097953, ARPIN N ET AL: "Chemotaxonomic studies on fungi. Fungal carotenoids. IV. Phillipsia carminea carotenoids. Isolation and identification of a new, naturally occurring xanthophyll" XP002002865 & BULL. SOC. CHIM. BIOL. (BSCIA3);67; VOL.49 (5); PP.527-361, FAC. SCI., LYON;C.N.R.S.; LYON; FR.,
- ACTA CHEM. SCAND. (ACSAA4);72; VOL.26 (6); PP.2528-30, UNIV. TRONDHEIM;NORTW. INST. TECHNOL.; TRONDHEIM; NORWAY, XP002002864 AGUILAR-MARTINEZ M ET AL: "Bacterial carotenoids. XL. 2'-Hydroxyflexixanthin"

## Beschreibung

Die vorliegende Erfindung betrifft ein neues Verfahren zur Herstellung von β-Ketoalkoholen aus Aldehyden durch eine besondere Kondensationsreaktion.

Chemistry Letters 1991, 1377-1378 (Inoue et al.) offenbart eine Cycloadditionsreaktion zwischen einem cyclischen α-Methylencarbonat, nämlich 4,4-Dimethyl-5-methylen-1,3-dioxolan-2-on (nachfolgend "Cyclocarbonat"), und einem aromatischen Aldehyd unter Abspaltung von Kohlendioxid zu einem 2,2-Dimethyl-5-aryldihydrofuran-3-on. Diese Umsetzung erfolgt unter Verwendung von verschiedenen, Palladium oder andere Uebergangsmetalle enthaltenden Katalysatoren. Mit Benzaldehyd als aromatischem Aldehyd gelingt die Reaktion auch in Gegenwart einer Lewis-Säure als Katalysator, z.B. Zink- oder Aluminiumchlorid, wobei allerdings auch 2-Methyl-3-oxo-5-phenylpent-4-en-2-ol [C₆H₅CH=CHCOC(CH₃)₂OH] und/oder ein anderes Nebenprodukt erhalten wird. In dieser Literaturstelle ist allerdings weder von der Verwendung einer Base anstelle des ein Uebergangsmetall enthaltenden Katalysators noch vom Einsatz anderer als aromatischer Aldehyde (ArCHO) die Rede.

Bull. Soc. Chim. Biol. 49(5), 527-536 (1967) / CA 67, 97953g (1967) berichtet über die Isolierung und Charakterisierung von sechs Carotinoiden aus Phillipsia carminea, u.a. von einem Ester von 2'-Dehydroplectaniaxanthin. 2'-Dehydroplectaniaxanthin weist die 4-Hydroxy-4-methyl-3-oxo-1-pentenyl-Endgruppe auf. Die Synthese von diesem und verwandten Carotinoiden ist allerdings nicht offenbart.

Acta Chem. Scand. 26, Nr. 6, 2528-2529 (1972) offenbart die Isolierung von Flexixanthin und dessen 2'-Hydroxyderivat von einem Bakterienstamm. Im Rahmen einer Struktur-Analyse wurde 2'-Hydroxyflexixanthin mit p-Chloranil zu 2'-Ketoflexixanthin oxidiert, welches ebenfalls die obenerwähnte Endgruppe aufweist.

Es wurde nun überraschenderweise gefunden, dass sich das obenerwähnte Reagens Cyclocarbonat oder ein Derivat davon sehr gut zur Ueberführung einer Formylgruppe -CHO, die sich in der Endstellung der konjugierten Kette eines gegebenenfalls substituierten konjugierten Polyenaldehyds befindet, in eine 4-Hydroxy-4-methyl-3-oxo-1-pentenylgruppe -CH=CH-CO-C(CH₃)₂OH bzw. ein entsprechendes Derivat eignet, wenn die Umsetzung des Aldehyds mit dem Cyclocarbonat oder Derivat davon unter basischen Bedingungen durchgeführt wird. Unter dem Begriff "gegebenenfalls substituierter konjugierter Polyenaldehyd" verstehen sich nicht nur diesbezügliche Monoaldehyde (A-CHO) sondern auch diesbezügliche Dialdehyde (OHC-B-CHO), wobei im letzteren Fall beide freien endständigen Formylgruppen in die 4-Hydroxy-4-methyl-3-oxo-1-pentenylgruppe oder ein entsprechendes Derivat übergeführt werden. Demnach handelt es sich bei der vorliegenden Erfindung um ein Verfahren zur Ueberführung eines gegebenenfalls substituierten konjugierten Polyenaldehyds der allgemeinen Formel

A-CHO I'

oder

OHC-B-CHO Iʺ

worin A oder B den gegebenenfalls substituierten konjugierten Polyen-Monoaldehyd bzw. -Dialdehyd ausser der Formylgruppe bzw. der beiden Formylgruppen darstellt und wobei sich die Formylgruppe(n) in der(den) Endstellung(en) der konjugierten Kette dieses Polyenaldehyds befindet bzw. befinden, in einen Polyen(di)alkohol der allgemeinen Formel

A-CH=CH-CO-CR¹R²OH II'

bzw.

HOR²R¹C-OC-HC=HC-B-CH=CH-CO-CR¹R²OH IIʺ

worin
- R¹: eine C₁₋₆-Alkylgruppe und
- R²: eine C₁₋₆-Alkylgruppe oder eine C₂₋₆-Alkenylgruppe bedeuten, oder
- R¹ und R²: zusammen 1,4-Tetramethylen oder 1,5-Pentamethylen bilden,
das dadurch gekennzeichnet ist, dass man den Polyenaldehyd unter basischen Bedingungen mit Cyclocarbonat oder einem Derivat davon der allgemeinen Formel 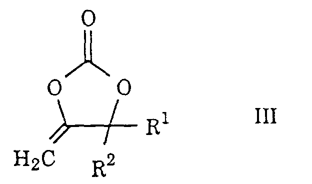 worin R¹ und R² die oben angegebenen Bedeutungen besitzen, umsetzt. Auf diese Weise wird die (jede) an eine konjugierte Doppelbindung gebundene endständige Formylgruppe des gegebenenfalls substituierten konjugierten Polyenaldehyds in eine Gruppe -CH=CH-CO-CR¹R²OH übergeführt.

Das erfindungsgemässe Verfahren kann im Prinzip bei allen obenerwähnten Polyenaldehyden A-CHO oder OHC-B-CHO, welche am Ende bzw. an beiden Enden der Polyenkette die Gruppierung -C=C-CHO, z.B. -CH=CH-CHO, -C(CH₃)=CH-CHO oder -CH=C(CH₃)-CHO, aufweisen, Verwendung finden. Unter solchen Polyenaldehyden (Edukten) befinden sich u.a. die folgenden Unterklassen [wobei die in der Carotinoid-Chemie übliche (mit einfachen Strichen) abgekürzte Darstellungsweise für die Strukturformeln benutzt wird]:
Alicyclisch-aliphatische Polyenaldehyde, die hauptsächlich dem Gebiet der Carotinoide [als asymmetrische Carotinoid-Aldehyde mit einem sechsgliedrigen (Cyclohexen-)Ring] angehören, der allgemeinen Formel 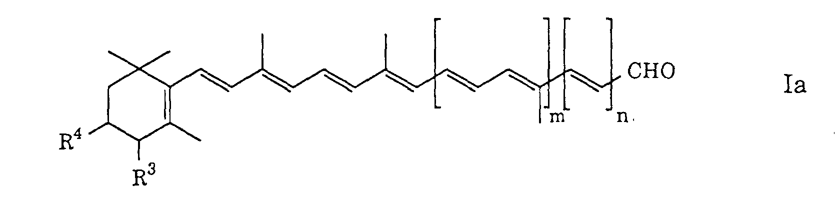 worin
   - R³ und R⁴: unabhängig voneinander Wasserstoff, eine gegebenenfalls geschützte Hydroxygruppe oder eine gegebenenfalls geschützte Oxogruppe,
   - m: 0, 1, 2, 3 oder 4
   und
   - n: 0 oder 1 bedeuten,
   welche nach Umsetzung mit der Verbindung der Formel III unter basischen Bedingungen in die entsprechenden alicyclisch-aliphatischen Polyenalkohole der allgemeinen Formel 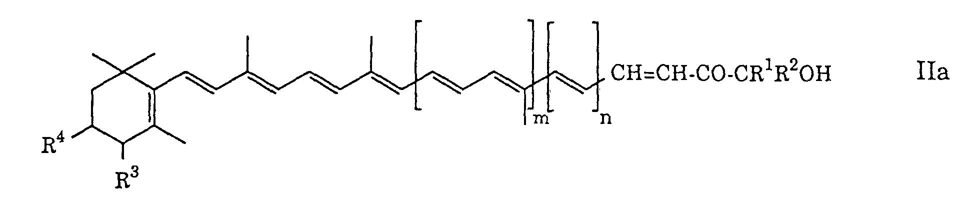 übergeführt werden;
Aliphatische Polyenaldehyde, die ebenfalls hauptsächlich dem Gebiet der Carotinoide (als offenkettige asymmetrische Carotinoid-Aldehyde) angehören, der allgemeinen Formel 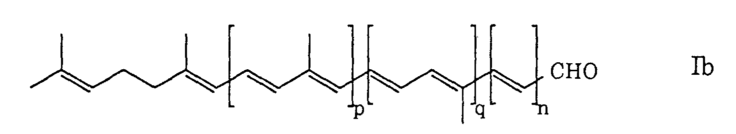 worin
   - p: 0, 1 oder 2,
   - q: 0, 1, 2 oder 3
   und
   - n: 0 oder 1 bedeuten,
   welche nach Umsetzung mit der Verbindung der Formel III unter basischen Bedingungen in die entsprechenden aliphatischen Polyenalkohole der allgemeinen Formel 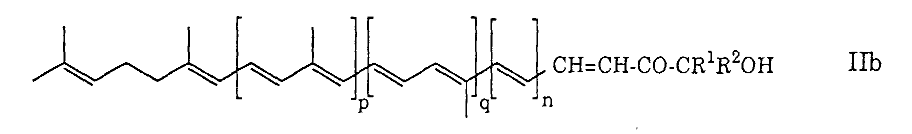 übergeführt werden;
Aliphatische Polyenaldehyde, die ebenfalls hauptsächlich dem Gebiet der Carotinoide (als symmetrische Carotinoid-Dialdehyde) angehören, der allgemeinen Formel 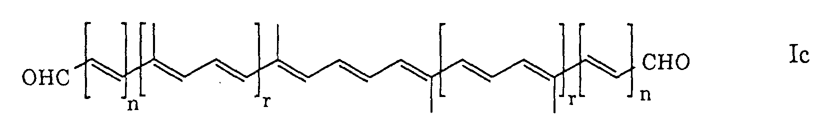 worin
   - r: 0, 1 oder 2
   und
   - n: 0 oder 1 bedeuten,
   und wobei eine der beiden Formylgruppen gegebenenfalls geschützt ist,
   welche nach Umsetzung mit der Verbindung der Formel III unter basischen Bedingungen in die entsprechenden aliphatischen Polyen-Dialkohole bzw. -Alkohole der allgemeinen Formel 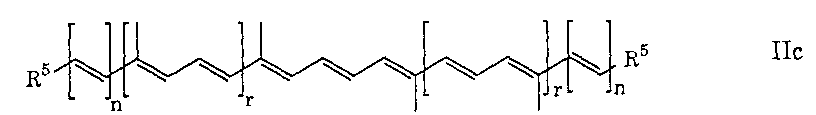 worin n und r die oben angegebenen Bedeutungen besitzen und die beiden R⁵ jeweils die Gruppe -CH=CH-CO-CR¹R²OH oder das eine R⁵ diese Gruppe und das andere eine geschützte Formylgruppe bedeuten,
übergeführt werden.

Die Edukte der allgemeinen Formeln Ia, Ib und Ic können durch die allgemeine Formel I umfasst werden:

R - CHO I

worin
- R: eine Gruppe (a), (b) oder (c) 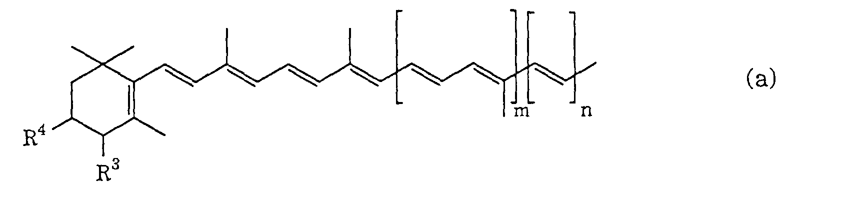 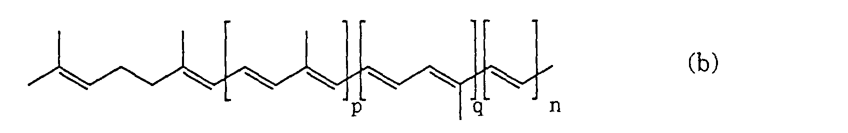 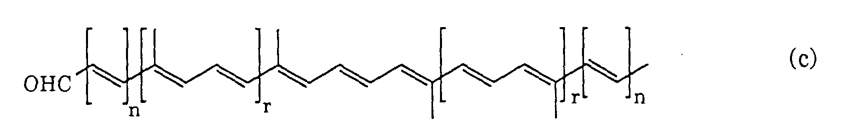 bedeutet und
- R³, R⁴, m, n, p, q und r: die oben angegebenen Bedeutungen besitzen,
wobei im Falle, dass R eine Gruppe (c) bedeutet, eine der beiden Formylgruppen gegebenenfalls geschützt ist.

Nach Umsetzung mit Cyclocarbonat oder einem Derivat davon der Formel III unter basischen Bedingungen wird das Edukt der Formel I in das entsprechende Produkt der Formel II übergeführt:

R'-CH=CH-CO-CR¹R²OH II

worin R' die oben angegebene Bedeutung von R hat, wobei, im Falle, dass R' eine Gruppe (c) bedeutet, die Formylgruppe entweder geschützt oder durch die Gruppe HOR²R¹C-CO-CH=CH- ersetzt ist.

Falls das Produkt der Formel II, insbesondere der Formel IIa oder IIc, eine oder mehrere Schutzgruppen aufweist, kann man gewünschtenfalls die vorhandene(n) Schutzgruppe(n) abspalten, was einen weiteren Aspekt der vorliegenden Erfindung darstellt.

Im Rahmen der vorliegenden Erfindung umfasst der Ausdruck "C₁₋₆-Alkylgruppe" oder "C₂₋₆-Alkenylgruppe" geradkettige und verzweigte Gruppen, wie beispielsweise Methyl, Aethyl und Isobutyl bzw. Vinyl oder 4-Methyl-3-pentenyl. Falls R¹ und R² zusammen 1,4-Tetramethylen oder 1,5-Pentamethylen bilden, bedeutet die diesbezügliche Gruppierung -CR¹R²- (in den Formeln II', II", III, IIa, IIb und IIc) Cyclopentyliden bzw. Cyclohexyliden.

Der Ausdruck "geschützte Hydroxygruppe" umfasst übliche (besonders auf dem Gebiet der Carotinoide geläufige) geschützte Hydroxygruppen, insbesondere verätherte Hydroxygruppen und Acyloxygruppen. Bei den "verätherten Hydroxygruppen" handelt es sich beispielsweise um C₁₋₅-Alkoxygruppen, vorzugsweise Methoxy und Aethoxy; C₂₋₁₆-Alkoxyalkoxygruppen, vorzugsweise 1-Methoxy-1-methyläthoxy; Arylalkoxygruppen, vorzugsweise Benzyloxy; Tetrahydropyranyloxy; und Tri(C₁₋₅-alkyl)silyloxygruppen, vorzugsweise Trimethylsilyloxy. Die Acyloxygruppen umfassen insbesondere Alkanoyloxy- und Aroyloxygruppen mit bis zu 8 Kohlenstoffatomen, wie beispielsweise Formyloxy, Acetoxy, Propionyloxy und Benzoyloxy.

Der Ausdruck "geschützte Oxogruppe" umfasst ebenfalls übliche (besonders auf dem Gebiet der Carotinoide geläufige) geschützte Oxogruppen. Bevorzugt sind die acetalisierten Oxogruppen, insbesondere diejenigen, worin der Ausdruck geschützte Oxogruppe für zwei C₁₋₅-Alkoxygruppen (z.B. für zwei Methoxygruppen) oder für eine C₂₋₆-Alkylendioxygruppe (z.B. Aethylendioxy oder 2,3-Butylendioxy) steht. Ferner kann eine Oxogruppe auch als Enoläther geschützt sein, und zwar vor allem im Falle von α-Hydroxyketonen (z.B. R³ und R⁴ bedeuten Hydroxy resp. Oxo oder umgekehrt), wobei die Verätherung des Endiols vorzugsweise auch durch Bildung eines cyclischen Acetals oder Ketals (z.B. mit Aceton zum Acetonid) erfolgen kann. Die Oxogruppe kann auch beispielsweise als ein Imin geschützt sein.

Im Falle des Einsatzes eines aliphatischen Polyenaldehyds der Formel Ic als Edukt, in welcher die eine Formylgruppe geschützt ist, liegt diese geschützte Formylgruppe zweckmässigerweise als acetalisierte Formylgruppe vor. Es handelt sich dabei um eine übliche acetalisierte Aldehydgruppe, insbesondere um eine Di(C₁₋₅-alkoxy)methylgruppe, z.B. Dimethoxymethyl, oder um 3-Dioxolan-2-yl oder ein analoges Kondensat der Formylgruppe mit einem C₂₋₆-Alkylendiol, z.B. 2,2-Dimethyltrimethylenglykol.

Falls nicht weiter qualifiziert umfasst der Ausdruck "Alkyl" geradkettige und verzweigte Gruppen, vorzugsweise mit 1-5 Kohlenstoffatomen, wie beispielsweise Methyl, Aethyl, n-Propyl, Isopropyl, tert.Butyl und dergleichen. Dies gilt auch für den Alkylteil von "Alkoxy". Ebenfalls umfasst der Ausdruck "Alkylen" geradkettige und verzweigte Gruppen, in diesem Fall insbesondere mit 2-6 Kohlenstoffatomen, z.B. 2,3-Butylen oder insbesondere Polymethylen, wie Aethylen, Trimethylen, Tetramethylen, Pentamethylen und Hexamethylen.

Falls eine Arylgruppe vorkommt, ist diese vorzugsweise Phenyl, welches gegebenenfalls, z.B. mit einer oder mehreren C₁₋₅-Alkyl- und/oder Nitrogruppen, substituiert ist. In der Regel ist jedoch unsubstituiertes Phenyl bevorzugt.

Die im Rahmen der vorliegenden Erfindung offenbarten Formeln von Polyenen umfassen jeweils isomere Formen, z.B. optisch aktive und cis/trans bzw. E/Z-Isomere, sowie Gemische hiervon, sofern nicht ausdrücklich anders erwähnt. Als Beispiel eines chiralen (optischen aktiven) Zentrums sei erwähnt das den Rest R³ oder R⁴ tragende Kohlenstoffatom, falls R³ bzw. R⁴ eine gegebenenfalls geschützte Hydroxygruppe bedeutet (siehe Formeln Ia und IIa). Was die E/Z-Isomerie anbelangt, so sind im allgemeinen die all-E-Isomeren der Edukte und der Produkte des erfindungsgemässen Verfahrens bevorzugt.

Vorzugsweise bedeutet sowohl R¹ als auch R² Methyl. In diesem Fall wird als Reagens der Formel III Cyclocarbonat selber verwendet, um die endständige(n) Formylgruppe(n) des Polyenaldehyds A-CHO bzw. OHC-B-CHO in die 4-Hydroxy-4-methyl-3-oxo-1-pentenylgruppe überzuführen.

Das erfindungsgemässe Verfahren wird zweckmässigerweise durchgeführt, indem man den Polyenaldehyd mit 1 bis 5 Aequivalenten des Cyclocarbonats bzw. Cyclocarbonatderivats der Formel III in einem organischen oder wässrig-organischen Lösungsmittel bei Temperaturen im Bereich von 25°C bis 120°C und in Gegenwart einer Base umsetzt. Als organische Lösungsmittel eignen sich im allgemeinen alle polaren protischen oder aprotischen Lösungsmittel, welche gegenüber der jeweils verwendeten Base inert und ausserdem mit Wasser mischbar sind. Besonders bevorzugte Lösungsmittel sind niedere Alkohole, insbesondere diejenigen mit 1 bis 3 Kohlenstoffatomen, z.B. Aethanol; cyclische Aether, z.B. Tetrahydrofuran und Dioxan; Aromaten, z.B. Toluol; sowie halogenierte niedere aliphatische Kohlenwasserstoffe, z.B. Methylenchlorid. Solche organischen Lösungsmittel können im Gemisch mit Wasser verwendet werden. Die verwendete Base kann anorganisch oder organisch sein, und es eignen sich insbesondere Alkalihydroxide, z.B. Natrium- und Kaliumhydroxide, und Alkalialkoxide, vorzugsweise die aus C₁₋₃-Alkanolen erzeugten, wie beispielsweise Natriumäthoxid. Insbesondere im Falle des Einsatzes eines Alkalihydroxids als Base wird ein wässrig-organisches Reaktionsmedium (Lösungsmittel) verwendet. Man verwendet zweckmässigerweise mindestens 2 Aequivalente Base pro Aequivalent Cyclocarbonat bzw. Cyclocarbonatderivat, vorzugsweise etwa 2,5 bis etwa 3,5 Aequivalente. Wie oben angedeutet, können die Reaktionstemperaturen in einem relativen breiten Bereich (von 25°C bis 120°C) variieren; die Umsetzung erfolgt allerdings vorzugsweise bei Temperaturen von 40°C bis 80°C. Ausserdem erfolgt die Umsetzung zweckmässigerweise bei Normaldruck, wobei im allgemeinen der Druck nicht kritisch ist.

Nach Beendigung der Reaktion kann die Base durch Zugabe einer organischen oder anorganischen Säure neutralisiert werden. Anschliessend kann je nach eingesetztem Polyenaldehyd das Produkt direkt durch Filtration oder sonst durch Extraktion und Abtrennung allfällig vorhandenen Salzes auf an sich bekannte Weise isoliert werden. Eine weitere Reinigung kann beispielsweise durch Destillation, Umkristallisation usw., d.h. nach an sich bekannten Methoden, erfolgen.

Speziell einfach gestaltet sich die Aufarbeitung bei Verwendung von Lösungsmitteln, die mit Wasser mischbar sind. Nach beendeter Reaktion und allfälliger Neutralisation der Base werden 10-200% (bevorzugt 50-150%) Wasser bezogen auf die Menge des eingesetzten organischen Lösungsmittels zugegeben. Bei Temperaturen zwischen Raumtemperatur und Siedepunkt wird dann kurzzeitig oder bei Bedarf auch mehrere Stunden gerührt. Hierbei werden nicht nur allfällig vorhandene Salze aus dem kristallinen Produkt ausgewaschen, sondern meist läuft auch die Umwandlung eines Z-Isomeren zum gewünschten all-E-Isomeren gleichzeitig ab. Das reine Produkt kann anschliessend durch einfaches Filtrieren, Waschen und Trocknen isoliert werden.

Im erhaltenen Produkt gegebenenfalls vorhandene Schutzgruppen können gewünschtenfalls ebenfalls nach an sich bekannten Methoden, z.B. durch Hydrolyse mit Säure oder Base, abgespalten werden.

Ein Teil der Edukte des erfindungsgemässen Verfahrens ist bekannt, und die Herstellung dieser bekannten Edukte ist in der Fachliteratur (hauptsächlich betreffend Carotinoide) gut dokumentiert. So sind beispielsweise die Umsetzung von verschiedenen C₁₅-Wittigsalzen mit 2,7-Dimethyl-2,4,6-octatriendial (dem sogenannten "C₁₀-Dialdehyd") zu den entsprechenden Monoaldehyden der oben angegebenen Formeln Ia und Ib, die Umsetzung von verschiedenen C₅-Wittigaldehyden mit langkettigen Polyenaldehyden ebenfalls zu solchen Monoaldehyden sowie die zweifache Umsetzung des C₁₀-Dialdehyds mit C₅- oder C₁₀-Wittigaldehyden zu verschiedenen Dialdehyden der oben angegebenen Formel Ic aus dieser Literatur bekannt geworden. Das Lehrbuch "Carotenoids" (O. Isler, Birkhäuser Verlag Basel und Stuttgart, 1971), besonders dessen Kapitel VI und XII und die darin erwähnte weitere Literatur, liefert viele nützliche Hinweise auf die Herstellung und das Vorkommen der bekannten Edukte. Falls Edukte eingesetzt werden, welche geschützte Hydroxy-, Oxo- bzw. Formylgruppen aufweisen, so können solche "geschützte" Edukte beispielsweise unmittelbar aus den entsprechenden ungeschützten nach an sich bekannten Methoden hergestellt werden.

Die neuen Edukte können auf analoge Weise zu den bekannten hergestellt werden.

Das Reagens 4,4-Dimethyl-5-methylen-1,3-dioxolan-2-on ("Cyclocarbonat" selber) ist eine bekannte Verbindung, deren Herstellung (leicht aus 2-Methyl-3-butin-2-ol) beispielsweise in der Deutschen Patentschrift 1.098.953 sowie in Synthesis 1981, 958-959, Bull. Chem. Soc. Japan 60, 1204-1206 (1987), Tetrahedron Lett. 30, 3981-3982 (1989) und weiteren Literaturstellen, auf welche in der eingangs erwähnten Publikation Chemistry Letters 1991, 1377-1378 (Inoue et al.) hingewiesen ist, beschrieben ist. Die Herstellung von sowohl Cyclocarbonat als auch Derivaten davon ist in den europäischen Patentpublikation 175.241 beschrieben.

Gewisse Endprodukte des erfindungsgemässen Verfahrens sind neue Verbindungen.

Die Endprodukte des erfindungsgemässen Verfahrens gehören grösstenteils dem Gebiet der Carotinoide an und finden entsprechende Verwendung, beispielsweise als Farbstoffe bzw. Pigmente für Lebensmittel, den Eidotter, die Integumente (insbesondere Haut, Ständer und Schnäbel) und/oder das subkutane Fett von Geflügel, das Fleisch und/oder die Integumente (insbesondere Haut, Schuppen und Schale) von Fischen und Crustaceen usw. Diese Verwendung kann nach an sich bekannten Methoden erfolgen, wie diese beispielsweise in der europäischen Patentpublikation Nr. 630.578 beschrieben ist.

Die Erfindung wird durch die folgenden Beispiele veranschaulicht:

### Beispiel 1

### Umsetzung von Vitamin A-Aldehyd mit Cyclocarbonat zu 13'-Hydroxy-13',14'-dihydro-12'-apo-β-carotin-14'-on

In einem mit Rührer, Thermometer, Kühler und Tropftrichter versehenen 200 ml-Sulfierkolben werden unter Argon 4,97 g (0,0175 Mol) Vitamin A-Aldehyd in 50 ml n-Propanol aufgeschlämmt. Zu der resultierenden gelben Lösung werden 7,82 g (0,060 Mol) Cyclocarbonat mit 20 ml n-Propanol dazugespült. Dann tropft man zu der gelben Lösung innert einer Minute 9,6 ml 50%ige Kaliumhydroxidlösung zu. Dabei steigt die Temperatur auf 51°C an. Die Lösung wird trüb und verfärbt sich zuerst dunkelorange, dann bräunlich. Das Gemisch wird in einem Oelbad auf 70°C erwärmt. Es lagert sich an der Kolbenwand ein weisser Niederschlag ab. Gemäss Dünnschichtchromatographie ist die Reaktion bereits nach einer Stunde fast beendet. Man kühlt nach weiteren zwei Stunden das orangebraune, trübe Reaktionsgemisch mittels eines Wasserbades auf Raumtemperatur ab.

Zwecks Aufarbeitung gibt man zum Gemisch 50 ml halbgesättigte Natriumchloridlösung und rührt es kräftig zwei Minuten lang. Anschliessend wird das Ganze mit 30 ml n-Propanol in einen 250 ml-Scheidetrichter gespült und die Phasen trennen gelassen. Man trennt die wässrige Phase ab und extrahiert die organische Phase dreimal mit jeweils 50 ml halbgesättigter Natriumchloridlösung, wobei die letzte Wasserphase einen pH-Wert von etwa 6 aufweist. Die vereinigten wässrigen Phasen werden dann zweimal mit jeweils 25 ml n-Propanol nachextrahiert. Nach Trocknung der vereinigten organischen Phasen über wasserfreiem Natriumsulfat werden diese unter vermindertem Druck bei 50°C vollständig eingeengt. Es bleibt ein orangerotes, zum Teil kristallines Oel zurück. Auf diese Weise erhält man das 13'-Hydroxy-13',14'-dihydro-12'-apo-β-carotin-14'-on (7,1 g) in rohem Zustand, wobei die Ausbeute des gewünschten Produktes vermutlich nahezu 100% beträgt.
¹H-NMR [CDCl₃, Tetramethylsilan (TMS) als interner Standard]: δ = 1,03 ppm (s,6H), 1,40 ppm (s,6H), 4,11 ppm (s,1H), 7,86-7,93 ppm (m,1H).

### Beispiel 2

### Umsetzung von β-Apo-12'-carotinal mit Cyclocarbonat zu 9'-Hydroxy-9',10'-dihydro-8'-apo-β-carotin-10'-on

In einem mit Rührer, Thermometer, Kühler und Tropftrichter versehenen 200 ml-Sulfierkolben werden unter Argon 7,01 g (0,020 Mol) β-Apo-12'-carotinal in 30 ml Aethanol aufgeschlämmt. Zu der resultierenden dunkelroten Suspension werden 7,82 g (0,060 Mol) Cyclocarbonat mit 20 ml Aethanol dazugespült und anschliessend innert 5 Minuten 10,3 ml 50%ige Kaliumhydroxidlösung zugetropft. Dabei wird zeitweise in einem Wasserbad leicht gekühlt, damit die Temperatur 50°C nicht überschreitet. Die orangebraune Suspension wird auf 50°C erwärmt. Es lagert sich an der Kolbenwand ein weisser Niederschlag ab, und schon nach kurzer Zeit erscheinen an der Kolbenwand auch orange Flecken. Die Reaktion wird mittels Dünnschichtchromatographie und HPLC verfolgt, wonach die Reaktion nach 3,5 Stunden praktisch beendet ist. Man kühlt die resultierende orangerote Suspension in einem Eis/Wasser-Bad auf 3°C ab und rührt bei dieser Temperatur noch eine Stunde. Dann wird filtriert und das Kristallisat dreimal mit jeweils 50 ml Wasser gewaschen und danach
16 Stunden unter vermindertem Druck bei 50°C getrocknet. Auf diese Weise erhält man 7,69 g 9'-Hydroxy-9',10'-dihydro-8'-apo-β-carotin-10'-on als rotes Kristallisat. Die Ausbeute entspricht etwa 88,5% der Theorie.
¹H-NMR (CDCl₃, TMS als interner Standard): δ = 1,04 ppm (s,6H), 1,42 ppm (s,6H), 1,72 ppm (s,3H), 1,96-2,02 ppm (m,11H), 4,10 ppm (s,1H), 6,12-6,29 ppm (m,6H), 6,61-6,88 ppm (m,4H), 7,54-7,58 ppm (d,1H).

### Beispiel 3

### Umsetzung von β-Apo-8'-carotinal mit Cyclocarbonat zu 5'-Hydroxy-5',6'-dihydro-4'-apo-β-carotin-6'-on

In einem mit Rührer, Thermometer, Kühler und Tropftrichter versehenen 350 ml-Sulfierkolben werden unter Argon 10,00 g (0,024 Mol) β-Apo-8'-carotinal in 80 ml Dioxan aufgeschlämmt. Zu der resultierenden dünnen Suspension werden 9,30 g (0,072 Mol) Cyclocarbonat mit 20 ml Dioxan dazugespült und anschliessend innert 20 Minuten 12,45 ml 50%ige Kaliumhydroxidlösung zugetropft. Man erhitzt dann das Gemisch mittels eines Wasserbads auf Rückflusstemperatur (etwa 100°C), und nach 22 Stunden sind gemäss Dünnschichtchromatographie und HPLC noch 3,8% Edukt im Reaktionsgemisch enthalten. Es werden etwa 50 ml des Dioxans bei Normaldruck abdestilliert, wonach das Oelbad entfernt wird. Man gibt 150 ml n-Propanol und 100 ml Wasser zum Gemisch zu, worauf bräunliche Kristalle ausfallen. Anschliessend wird das Reaktionsgemisch wieder auf Rückflusstemperatur (ca. 80°C) erhitzt und 30 Minuten bei dieser Temperatur gerührt, danach auf Raumtemperatur abgekühlt und 15 Minuten bei dieser Temperatur nachgerührt. Nach Abfiltration der violetten Kristalle werden diese zweimal mit jeweils 50 ml n-Propanol und anschliessend dreimal mit jeweils 50 ml Wasser nachgewaschen, schliesslich 16 Stunden unter vermindertem Druck (20 mbar) bei etwa 60°C getrocknet. Auf diese Weise erhält man 6,88 g (Ausbeute etwa 57,2% der Theorie) 5'-Hydroxy-5',6'-dihydro-4'-apo-β-carotin-6'-on als feine violette Kristalle.
¹H-NMR (CDCl₃, TMS als interner Standard): δ = 1,03 ppm (s,6H), 1,43 ppm (s,6H), 1,46-1,48 ppm (m,2H), 1,60-1,63 ppm (m,2H), 1,72 ppm (s,3H), 1,98-2,04 ppm (m,14H), 4,09 ppm (s breit, 1H), 6,14-6,73 ppm (m,13H), 7,55+7,59 ppm (d,1H).

### Beispiel 4

### Umsetzung von β-Apo-4'-carotinal mit Cyclocarbonat zu 2'-Dehydroplectaniaxanthin

In einem mit Rührer, Thermometer, Kühler und Dosierpumpe versehenen 350 ml-Sulfierkolben werden unter Argon 10,12 g β-Apo-4'-carotinal in 50 ml n-Propanol aufgeschlämmt. Dann werden 9,6 ml 50%ige Kalilauge zugegeben. Die rotviolette, dünne Suspension wird nun auf 70°C Innentemperatur erwärmt. Dann wird mittels der Dosierpumpe innert einer Stunde bei 70°C eine Lösung von 7,72 g Cyclocarbonat in 20 ml n-Propanol zudosiert. Man rührt das Gemisch unter Dünnschichtchromatographie- und HPLC-Kontrolle 5 Stunden bei 70°C. Nach dieser Zeit ist gemäss HPLC praktisch das ganze β-Apo-4'-carotinal umgesetzt. Man gibt zum Gemisch 70 ml Wasser zu, erhitzt das rotbraune, dünne Reaktionsgemisch zum Rückfluss und rührt es bei einer Innentemperatur von 89,3°C. Danach wird das Oelbad entfernt, und das Gemisch mittels eines Wasserbads auf 22°C abgekühlt und 30 Minuten gerührt. Die Kristalle werden anschliessend abgenutscht und nacheinander zweimal mit jeweils 50 ml n-Propanol und dreimal mit jeweils 50 ml Wasser gewaschen. Nach Abfiltration der Kristalle werden diese 16 Stunden bei 50°C unter vermindertem Druck getrocknet. Auf diese Weise erhält man 11,09 g (97,8% der Theorie) 2'-Dehydroplectaniaxanthin als braune Kristalle.
¹H-NMR (CDCl₃, TMS als interner Standard): δ = 1,03 ppm (s,6H), 1,43 ppm (s,6H), 1,72 ppm (s,3H), 1,98-2,03 ppm (m,17H), 4,14 ppm (s,1H), 6,11-6,24 ppm (m,3H), 6,27-6,47 ppm (m,6H), 7,55+7,59 ppm (d,1H).

### Beispiel 5

### Umsetzung von Torularhodinaldehyd mit Cyclocarbonat zu (all-E)-2-Hydroxy-2,6,10,14,18,23,27-heptamethyl-29-(2,6,6-trimethyl-cyclohex-1-enyl)-nonacosa-4,6,8,10,12,14,16,18,20,22,24,26,28-tridecaen-3-on

In einem mit Rührer, Thermometer, Kühler und Tropftrichter versehen 750 ml-Sulfierkolben werden unter Argon zu einem Gemisch von 20,00 g Torularhodinaldehyd und 18,79 g Cyclocarbonat in 200 ml n-Propanol 24,3 ml 50%ige wässrige Kaliumhydroxidlösung innert einer Minute unter Rühren zugetropft, wobei die Temperatur auf 50°C ansteigt. Man erhitzt das Reaktionsgemisch auf Rückflusstemperatur (etwa 90°C) und verfolgt den Reaktionsablauf mittels HPLC. Nach 2,5 Stunden wird festgestellt, dass noch 1,8% Edukt vorhanden ist, so dass die Reaktion durch Zugabe von 200 ml Wasser vorläufig abgebrochen wird und man anschliessend eine weitere Stunde auf Rückflusstemperatur (etwa 90°C) erhitzt. Danach wird die resultierende Suspension auf 25°C abgekühlt, und das Kristallisat abfiltriert und der Reihe nach zweimal mit jeweils 100 ml n-Propanol und dreimal mit jeweils 100 ml Wasser gewaschen. Man trocknet das resultierende schwarze Kristallisat 16 Stunden bei 50°C und unter vermindertem Druck. Es ergeben sich 21,7 g rohes Produkt.

Zwecks Reinigung des rohen Kristallisats löst man 19,31 g davon unter Rühren bei 25°C in 380 ml Methylenchlorid. Zur Lösung tropft man dann innert einer Stunde unter Rühren 380 ml Methanol zu, wobei glänzend dunkelviolette Kristalle ausfallen. Nach einstündigem Rühren bei 25°C werden die Kristalle abfiltriert und zweimal mit jeweils 60 ml Methanol/ Methylenchlorid (2:1) gewaschen. Man reinigt die Kristalle ein zweites Mal auf diese Weise und trocknet sie anschliessend 16 Stunden bei 50°C und unter vermindertem Druck. Auf diese Weise erhält man 14,7 g (63,7% der Theorie) (all-E)-2-Hydroxy-2,6,10,14,18,23,27-heptamethyl-29-(2,6,6-trimethylcyclohex-1-enyl)-nonacosa-4,6,8,10,12,14,16,18,20,22,24,26,28-tridecaen-3-on als dunkelbraune Kristalle.
¹H-NMR (CDCl₃, TMS als interner Standard): δ = 1,03 ppm (s,6H), 1,43 ppm (s,6H), 1,44-1,48 ppm (m,2H), 1,58-1,65 ppm (m,2H), 1,72 ppm (s,3H), 2,00-2,04 ppm (m,20H), 4,11 ppm (s breit, 1H), 6,11-6,17 ppm (m,3H), 6,21-6,49 ppm (m,8H), 6,58-6,70 ppm (m,7H), 7,55+7,59 ppm (d,1H).

### Beispiel 6

### Umsetzung von β-Apo-10'-carotinal mit Cyclocarbonat zu 7'-Hydroxy-7'-methyl-7',8'-dihydro-9'-nor-6'-apo-β-carotin-8'-on

In einem mit Rührer, Thermometer, Kühler und Tropftrichter versehen 350 ml-Sulfierkolben werden unter Argon 7,53 g (0,020 Mol) β-Apo-10'-carotinal in 50 ml n-Propanol aufgeschlämmt. Zu der resultierenden dunkelrotbraunen Suspension werden 7,82 g (0,060 Mol) Cyclocarbonat mit 20 ml n-Propanol dazugespült und anschliessend innert 2 Minuten 9,6 ml 50%ige Kaliumhydroxidlösung zugetropft. Dabei steigt die Temperatur auf 46°C an. Man erwärmt das Reaktionsgemisch auf 70°C, wonach sich an der Kolbenwand ein weisser Niederschlag ablagert. Gemäss Dünnschichtchromatographie ist nach 2,5 Stunden die Umsetzung fast beendet. Mittels Eisbadkühlung wird das Gemisch auf 0°C abgekühlt, und man rührt es anschliessend 3 Stunden. Dann werden die resultierenden Kristalle abgenutscht und der Reihe nach mit 50 ml n-Propanol und dreimal mit jeweils 50 ml Wasser gewaschen. Schliesslich trocknet man das Kristallisat etwa 16 Stunden bei 50°C und unter vermindertem Druck. Auf diese Weise erhält man 5,83 g (63,3% der Theorie) 7'-Hydroxy-7'-methyl-7',8'-dihydro-9'-nor-6'-apo-β-carotin-8'-on als weinrotviolette Kristalle.
¹H-NMR (CDCl₃, TMS als interner Standard): δ = 1,03 ppm (s,6H), 1,41 ppm (s,6H), 1,45-1,48 ppm (m,2H), 1,60-1,63 ppm (m,2H), 1,72 ppm (s,3H), 1,95-2,02 ppm (m,11H), 6,12-6,49 ppm (m,8H), 6,58-6,78 ppm (m,4H), 7,53-7,59 ppm (m, 1H).

### Beispiel 7

### Umsetzung von Crocetindialdehyd mit Cyclocarbonat zu 5,5'-Dihydroxy-5,6,5',6'-tetrahydro-4,4'-diapo-ψ,ψ-carotin-6,6'-dion

In einem mit Rührer, Thermometer, Kühler und Tropftrichter versehenen 200 ml-Sulfierkolben werden unter Argon 5,04 g (0,0170 Mol) Crocetindialdehyd in 20 ml n-Propanol aufgeschlämmt. Zu der resultierenden orangeroten Suspension werden 13,15 g (0,102 Mol) Cyclocarbonat mit 5 ml n-Propanol dazugespült und anschliessend innert 15 Minuten 16,26 ml 50%ige Kaliumhydroxidlösung zugetropft. Dabei steigt die Temperatur bis auf etwa 68°C. Es wird zeitweise in einem Eisbad leicht gekühlt, damit die Temperatur 70°C nicht überschreitet. Während der Zugabe der Kaliumhydroxidlösung ändert sich die Farbe des Gemisches von orangerot zu rot. Danach wird auf 70°C erwärmt und bei dieser Temperatur noch weitere 1,25 Stunden gerührt. Man fügt nach der Nachreaktion 50 ml Wasser hinzu und rührt das Reaktionsgemisch bei 70°C eine weitere Stunde nach. Das Gemisch wird wieder auf Raumtemperatur abgekühlt und der resultierende Kristallbrei genutscht und zweimal mit jeweils 15 ml
n-Propanol und dreimal mit jeweils 20 ml Wasser gewaschen. Man trocknet die rotvioletten Kristalle 16 Stunden bei 50°C unter vermindertem Druck bei 20-30 mbar. Auf diese Weise erhält man 7,07 g (89,5% der Theorie) 5,5'-Dihydroxy-5,6,5',6'-tetrahydro-4,4'-diapo-ψ,ψ-carotin-6,6'-dion als rotviolette Kristalle.
¹H-NMR (CDCl₃, TMS als interner Standard): δ = 1,43 ppm (s,12H), 1,99 ppm (s,12H), 4,10 ppm (s,2H), 6,38-6,73 ppm (m,12H), 7,54+7,58 ppm (d,2H).

### Beispiel 8

### Umsetzung von Diapo-4,4'-carotindial mit Cyclocarbonat zu Phillipsiaxanthin

In einem mit Rührer, Thermometer, Kühler und Tropftrichter versehenen 100 ml-Sulfierkolben werden unter Argon 3,00 g (0,0070 Mol) Diapo-4,4'-carotindial in 20 ml n-Propanol aufgeschlämmt. Zu der resultierenden orangeroten Suspension werden 5,41 g (0,042 Mol) Cyclocarbonat mit 5 ml n-Propanol dazugespült und anschliessend innert 15 Minuten 6,70 ml 50%ige Kaliumhydroxidlösung zugetropft. Dabei steigt die Temperatur bis auf etwa 68°C. Es wird zeitweise in einem Eisbad leicht gekühlt, damit die Temperatur 70°C nicht überschreitet. Während der Zugabe der Kaliumhydroxidlösung ändert sich die Farbe des Gemisches von rot zu rotbraun. Danach wird auf 70°C mittels eines Oelbads erwärmt und bei dieser Temperatur noch etwa 23 Stunden gerührt, währenddessen gemäss Dünnschichtchromatographie und HPLC die Umsetzung noch nicht beendet ist. Man entfernt das Oelbad und kühlt das Reaktionsgemisch auf etwa 50°C. Anschliessend werden zum Reaktionsgemisch noch 1,80 g (0,014 Mol) Cyclocarbonat sowie 2,32 ml 50%ige Kaliumhydroxidlösung innert 2 Minuten zugegeben, wobei die Temperatur des Reaktionsgemisches wieder auf etwa 65°C steigt. Man rührt das Ganze noch 1,25 Stunden bei 70°C, gibt 50 ml Wasser zu, und rührt bei 70°C eine weitere Stunde. Das Gemisch wird wieder auf Raumtemperatur abgekühlt und der resultierende rotbraune Kristallbrei genutscht und zweimal mit jeweils 15 ml n-Propanol und dreimal mit jeweils 20 ml Wasser gewaschen. Man trocknet die dunkelvioletten Kristalle 16 Stunden bei 50°C unter vermindertem Druck bei 20-30 mbar. Auf diese Weise erhält man 3,22 g (76,8% der Theorie) Phillipsiaxanthin als dunkelviolette Kristalle.
¹H-NMR (CDCl₃, TMS als interner Standard): δ = 1,43 ppm (s,12H), 1,99 ppm (s,12H), 2,01 ppm (s,6H), 4,12 ppm (s,2H), 6,32-6,48 ppm (m,8H), 6,59-6,69 ppm (m,10H), 7,54+7,58 ppm (d,2H).

### Beispiel 9

### Umsetzung von β-Apo-12'-canthaxanthinal mit Cyclocarbonat zu 2-Hydroxy-2,6,11,15-tetramethyl-17-(2,6,6-trimethyl-3-oxo-cyclohex-1-enyl)-heptadeca-4,6,8,10,12,14,16-heptaen-3-on

In einem mit Rührer, Thermometer, Kühler und Tropftrichter versehenen 100 ml-Sulfierkolben werden unter Argon 1,50 g (0,0041 Mol) β-Apo-12'-canthaxanthinal in 15 ml Methanol aufgeschlämmt. Zu der resultierenden Suspension werden 1,59 g (0,0124 Mol) Cyclocarbonat mit 6 ml Methanol dazugespült und anschliessend innert 3 Minuten unter Rühren 5,26 ml 30%ige Natriummethylatlösung zugetropft. Dabei steigt die Temperatur langsam auf 35°C an. Das Reaktionsgemisch wird in einem Oelbad auf 50°C erwärmt und unter Dünnschichtchromatographie- und HPLC-Kontrolle nachgerührt. Nach 5 Stunden bei 50°C wird die resultierende rote Suspension auf Rückflusstemperatur (65°C) erwärmt, wobei die Suspension dunkelrot und relativ dünn wird. Man rührt sie 2,5 Stunden bei der Rückflusstemperatur nach, kühlt sie anschliessend auf Raumtemperatur ab und rührt die wieder rot gewordene Suspension 16 Stunden bei dieser Temperatur nach. 29 ml Wasser werden dann zugegeben, und das Gemisch wird weitere 5 Stunden bei der Rückflusstemperatur (78°C) unter stetigem Rühren erhitzt. Dabei entsteht eine hellrote Suspension. Diese wird dann auf Raumtemperatur abgekühlt, eine Stunde nachgerührt und schliesslich filtriert, und das auf diese Weise isolierte rohe Reaktionsprodukt wird zweimal mit jeweils 2 ml Methanol bei -20°C, gefolgt von dreimal mit jeweils 2 ml Wasser, gewaschen und etwa 16 Stunden bei 45°C unter vermindertem Druck (Vakuumtrockenschrank) getrocknet.

Auf diese Weise erhält man 1,56 g 2-Hydroxy-2,6,11,15-tetramethyl-17-(2,6,6-trimethyl-3-oxo-cyclohex-1-enyl)-heptadeca-4,6,8,10,12,14,16-heptaen-3-on als rote Kristalle. Die Ausbeute entspricht etwa 84,4% der Theorie. ¹H-NMR (CDCl₃, TMS als interner Standard): δ = 1,20 ppm (s,6H), 1,43 ppm (s,6H), 1,84-1,87 ppm (m,5H), 1,98 ppm (s,3H), 2,02 ppm (s,6H), 2,49-2,53 ppm (t,2H), 4,09 ppm (s,1H), 6,25-6,50 ppm (m,6H), 6,64-6,90 ppm (m,4H), 7,54+7,58 ppm (d,1H).

### Beispiel 10

### Umsetzung von β-Apo-12'-zeaxanthinal mit Cyclocarbonat zu 2-Hydroxy-17-(4R-hydroxy-2,6,6-trimethyl-cyclohex-1-enyl)-2,6,11,15-tetramethyl-heptadeca-4,6,8,10,12,14,16-heptaen-3-on

In einem mit Rührer, Thermometer und Kühler versehenen 100 ml-Sulfierkolben werden unter Argon 1,83 g (0,0050 Mol) β-Apo-12'-zeaxanthinal in 15 ml Isopropanol aufgeschlämmt. Zu der resultierenden Suspension werden 1,93 g Cyclocarbonat mit 10 ml Isopropanol dazugespült und anschliessend bei 15°C mittels einer Pipette 2,58 ml 50%ige Kaliumhydroxidlösung so zugetropft, dass die Temperatur 22°C nicht übersteigt. Nach 18-stündiger Reaktion bei Raumtemperatur enthält das Reaktionsgemisch (rote Suspension) immer noch relativ viel Edukt, so dass weitere 1,29 g Cyclocarbonat (insgesamt dann 0,0250 Mol) zusammen mit wenig Isopropanol zugegeben werden. Wiederum bei einer Temperatur des Reaktionsgemischs von 15°C werden 1,47 ml 50%ige Kaliumhydroxidlösung so zugetropft, dass die Temperatur 22°C nicht übersteigt. Nach weiterer 2,75-stündiger Reaktion bei Raumtemperatur, wonach die Menge nichtreagierten Edukts nicht mehr abnimmt, gibt man innert einer Minute 35 ml entionisiertes Wasser zu. Dabei steigt die Temperatur auf etwa 27°C. Es fallen orange Kristalle aus. Die Suspension wird noch anderthalb Stunden bei Raumtemperatur gerührt, anschliessend abgenutscht und das erhaltene Kristallisat zweimal mit jeweils 6 ml Isopropanol bei 0°C und dreimal mit jeweils 6 ml entionisiertem Wasser gewaschen. Schliesslich werden die Kristalle etwa 16 Stunden bei 50°C unter vermindertem Druck (Vakuumtrockenschrank) getrocknet.

Auf diese Weise erhält man 1,51 g 2-Hydroxy-17-(4R-hydroxy-2,6,6-trimethyl-cyclohex-1-enyl)-2,6,11,15-tetramethyl-heptadeca-4,6,8,10,12,14,16-heptaen-3-on als orange Kristalle. Die Ausbeute entspricht etwa 67,0% der Theorie.
¹H-NMR (CDCl₃, TMS als interner Standard): δ = 1,08 ppm (s,6H), 1,42-1,51 ppm (m,7H), 1,74-1,79 ppm (m,4H), 1,97-2,08 ppm (m,10H), 2,36-2,42 ppm (m,2H), 4,00 ppm (m,1H), 4,10 ppm (s,1H), 6,14-6,44 ppm (m,6H), 6,58-6,88 ppm (m,4H), 7,54+7,58 ppm (d,1H).

### Beispiel 11

### Umsetzung von β-Apo-4'-zeaxanthinal mit Cyclocarbonat zu 2-Hydroxy-25-(4R-hydroxy-2,6,6-trimethyl-cyclohex-1-enyl)-2,6,10,14,19,23-hexamethylpentacosa-4,6,8,10,12,14,16,18,20,22,24-undecaen-3-on

In einem mit Rührer, Thermometer und Kühler versehenen 100 ml-Sulfierkolben werden unter Argon 1,78 g (0,0036 Mol) β-Apo-4'-zeaxanthinal in 15 ml Aethanol aufgeschlämmt. Zu der resultierenden Suspension werden 1,38 g (0,0107 Mol) Cyclocarbonat mit 10 ml Aethanol dazugespült und anschliessend innert einer Minute 1,84 ml 50%ige Kaliumhydroxidlösung zugetropft. Dabei steigt die Temperatur auf etwa 44°C. Mittels eines Oelbads wird das Reaktionsgemisch auf Rückflusstemperatur (78°C) erhitzt und unter Dünnschichtchromatographie- und HPLC-Kontrolle 4 Stunden nachgerührt. Dann werden innert einer Minute 35 ml Wasser zugegeben, wobei die Temperatur auf etwa 53°C sinkt und grobe rotbraune Kristalle ausfallen. Man erhitzt das Gemisch noch eine Stunde bei Rückflusstemperatur (etwa 81°C), um das Produkt isomerisieren zu lassen. Anschliessend wird das Oelbad durch ein Wasserbad ersetzt und das Gemisch auf Raumtemperatur abgekühlt. Nach etwa einstündigem Rühren werden die Kristalle abgenutscht und zweimal mit jeweils 6 ml Aethanol bei 0°C und dreimal mit jeweils 6 ml Wasser gewaschen. Schliesslich werden die erhaltenen dunkelbraunen Kristalle etwa 16 Stunden bei 50°C unter vermindertem Druck (Vakuumtrockenschrank) getrocknet.

Auf diese Weise erhält man 1,80 g 2-Hydroxy-25-(4R-hydroxy-2,6,6-trimethyl-cyclohex-1-enyl)-2,6,10,14,19,23-hexamethyl-pentacosa-4,6,8,10,12,14,16,18,20,22,24-undecaen-3-on als dunkelbraune Kristalle. Die Ausbeute entspricht etwa 86,5% der Theorie.
¹H-NMR (CDCl₃, TMS als interner Standard): δ = 1,08 ppm (s,6H), 1,43-1,51 ppm (m,8H), 1,74-1,79 ppm (m,4H), 1,98-2,08 ppm (m,16H), 2,36-2,42 ppm (m,1H), 3,99 ppm (m,1H), 4,13 ppm (s,1H), 6,13-6,17 ppm (m,3H), 6,25-6,48 ppm (m,6H), 6,59-6,71 ppm (m,7H), 7,55+7,58 ppm (d,1H).

### Beispiel 12

### Umsetzung von Apo-12'-lycopenal mit Cyclocarbonat zu (4E,6E,8E,10E,12E, 14E,16E,18E)-2-Hydroxy-2,6,11,15,19,23-hexamethyl-tetracosa-4,6,8,10,12,14, 16,18,22-nonaen-3-on

In einem mit Rührer, Thermometer, Kühler und Tropftrichter versehenen 500 ml-Sulfierkolben werden unter Argon 14,20 g (0,0405 Mol) Apo-12'-lycopenal in 50 ml Propanol aufgeschlämmt. Zu der resultierenden Suspension werden 10,44 g (0,081 Mol) Cyclocarbonat mit 72 ml Propanol dazugespült und anschliessend innert einer Minute 15,0 ml 50%ige Kaliumhydroxidlösung unter Rühren zugetropft. Dabei steigt die Temperatur auf etwa 42°C an. Mittels eines Oelbads wird dann das Reaktionsgemisch auf 70°C erhitzt und unter Dünnschichtchromatographie- und HPLC-Kontrolle nachgerührt. Nach einer Reaktionszeit bei 70°C von etwa 2 Stunden werden 170 ml Wasser zugegeben. Die resultierende orange-braune, glänzende Suspension wird wieder auf 70°C erhitzt und eine weitere Stunde gerührt. Anschliessend wird das Gemisch in einem Wasserbad auf etwa 25°C abgekühlt, 2 Stunden nachgerührt und über eine Glässinternutsche filtriert. Man presst den Kristallkuchen fest, wäscht ihn der Reihe nach zweimal mit jeweils 35 ml Propanol und dreimal mit jeweils 35 ml Wasser, wobei zwischen den Waschvorgängen abgesogen und der Kristallkuchen festgepresst wird, und trocknet ihn etwa 16 Stunden bei 50°C unter vermindertem Druck (Vakuumtrockenschrank).

Auf diese Weise erhält man 14,81 g (4E,6E,8E,10E,12E,14E,16E,18E)-2-Hydroxy-2,6,11,15,19,23-hexamethyl-tetracosa-4,6,8,10,12,14,16,18,22-nonaen-3-on als kupfer-orange, glänzende Kristalle. Die Ausbeute entspricht etwa 84,1% der Theorie.
¹H-NMR (CDCl₃, TMS als interner Standard): δ = 1,42 ppm (s,6H), 1,62 ppm (s,3H), 1,69 ppm (s,3H), 1,83 ppm (s,3H), 1,97 ppm (s,3H), 1,98 ppm (s,3H), 2,02 ppm (s,3H), 2,12 ppm (s,4H), 4,11 ppm (s,1H), 5,11 ppm (s,1H), 5,94+5,97 ppm (d,1H), 6,18-6,84 ppm (m,10H), 7,54+7,58 ppm (d,1H).

### Beispiel 13

### Umsetzung von Apo-4'-lycopenal mit Cyclocarbonat zu all-E-3,4-Didehydro-1,2-dihydro-1-hydroxy-ψ,ψ-carotin-2-on

In einem mit Rührer, Thermometer, Kühler und Tropftrichter versehenen 500 ml-Sulfierkolben werden unter Argon 13,50 g (0,028 Mol) Apo-4'-lycopenal in 80 ml Propanol aufgeschlämmt. Zu der resultierenden Suspension werden 10,81 g (0,084 Mol) Cyclocarbonat mit 60 ml Propanol dazugespült und anschliessend innert 3 Minuten 14,5 ml 50%ige Kaliumhydroxidlösung unter Rühren zugetropft. Dabei steigt die Temperatur auf etwa 43°C an. Mittels eines Oelbads wird dann das Reaktionsgemisch auf Rückflusstemperatur (93°C) erhitzt und unter Dünnschichtchromatographie- und HPLC-Kontrolle nachgerührt. Nach einer Reaktionszeit bei der Rückflusstemperatur von etwa 2 Stunden werden 200 ml Wasser zugegeben. Die resultierende braune Suspension wird wieder auf Rückflusstemperatur (88°C) erhitzt und eine weitere Stunde gerührt. Anschliessend wird das Gemisch auf etwa 25°C abgekühlt, 2,5 Stunden nachgerührt und über eine Glassinternutsche filtriert. Man presst den Kristallkuchen fest, wäscht ihn der Reihe nach zweimal mit jeweils 70 ml Propanol und dreimal mit jeweils 70 ml Wasser, wobei zwischen den Waschvorgängen abgesogen und der Kristallkuchen festgepresst wird, und trocknet ihn etwa 16 Stunden bei 50°C unter vermindertem Druck (Vakuumtrockenschrank).

Auf diese Weise erhält man 15,00 g all-E-3,4-Didehydro-1,2-dihydro-1-hydroxy-ψ,ψ-carotin-2-on als braune Kristalle. Die Ausbeute entspricht 94,6% der Theorie, allerdings bedarf das Produkt einer weiteren Reinigung.

Zwecks Reinigung werden 14,78 g des Produktes mit 150 ml Methylenchlorid unter Rühren aufgeschlämmt. Dann erhitzt man die resultierende Suspension in einem Oelbad auf Rückflusstemperatur und rührt sie 15 Minuten bei dieser Temperatur. Die Kristalle lösen sich nicht. Die Suspension wird in einem Wasserbad auf Raumtemperatur abgekühlt, eine Stunde bei dieser Temperatur gerührt und über eine Glassinternutsche genutscht. Man wäscht die erhaltenen Kristalle zweimal mit jeweils 25 ml Methylenchlorid und trocknet die resultierenden schwarzen, glänzenden Kristalle etwa 16 Stunden bei 50°C unter vermindertem Druck (Vakuumtrockenschrank).

Auf diese Weise erhält man 12,77 g (81,8% der Theorie) reineres all-E-3,4-Didehydro-1,2-dihydro-1-hydroxy-ψ,ψ-carotin-2-on als schwarze Kristalle.
¹H-NMR (CDCl₃, TMS als interner Standard): δ = 1,43 ppm (s,6H), 1,61 ppm (s,3H), 1,69 ppm (s,3H), 1,82 ppm (s,3H), 1,98 ppm (s,12H), 2,00 ppm (s,3H), 2,12 ppm (s,4H), 4,12 ppm (s,1H), 5,11 ppm (s,1H), 5,94+5,97 ppm (d,1H), 6,20-6,65 ppm (m,16H), 7,55+7,59 ppm (d,1H).

### Beispiel 14

### Umsetzung von β-Apo-4'-carotinal mit 4-Aethyl-4-methyl-5-methylen-1,3-dioxolan-2-on zu all-E-(R/S)-3-Hydroxy-3,7,11,15,20,24-hexamethyl-26-(2,6,6-trimethyl-cyclohex-1-enyl)-hexacosa-5,7,9,11,13,15,17,19,21,23,25-undecaen-4-on

In einem mit Rührer, Thermometer, Kühler und Tropftrichter versehenen 100 ml-Sulfierkolben werden unter Argon 4,96 g (0,01 Mol) β-Apo-4'-carotinal in 20 ml Isopropanol aufgeschlämmt. Zu der resultierenden Suspension werden 5,68 g (0,040 Mol) 4-Aethyl-4-methyl-5-methylen-1,3-dioxolan-2-on mit 5 ml Isopropanol dazugespült und anschliessend innert 10 Minuten 6,47 ml 50%ige Kaliumhydroxidlösung unter Rühren zugetropft. Dabei steigt die Temperatur auf etwa 57°C an. Die blutrote Suspension wird dann mittels eines Oelbads auf 80°C erhitzt, währenddessen die Farbe auf kupfrig wechselt. Nach einer gesamten Reaktionszeit von 4,5 Stunden werden dem Ansatz 25 ml Wasser zugegeben. Man erhitzt die braun-rote Suspension wieder auf 80°C und rührt sie eine weitere Stunde bei dieser Temperatur. Anschliessend wird mittels eines Wasserbads auf Raumtemperatur (21°C) abgekühlt und 30 Minuten bei dieser Temperatur nachgerührt. Dann nutscht man die glänzenden, grauen Kristalle ab, wäscht sie zweimal mit jeweils 25 ml Isopropanol und dreimal mit jeweils 25 ml Wasser und trocknet sie etwa 16 Stunden bei 50°C unter vermindertem Druck (Vakuumtrockenschrank).

Auf diese Weise erhält man 5,35 g all-E-(R/S)-3-Hydroxy-3,7,11,15,20,24-hexamethyl-26-(2,6,6-trimethyl-cyclohex-1-enyl)-hexacosa-5,7,9,11,13,15, 17,19,21,23,25-undecaen-4-on als violette Kristalle. Die Ausbeute entspricht etwa 92,1% der Theorie, allerdings bedarf das Produkt einer weiteren Reinigung; diese erfolgt durch mehrfache Umkristallisation aus einem Gemisch von Methylenchlorid und Methanol, wobei das noch vorhandene Edukt jeweils abgetrennt wird.
¹H-NMR (CDCl₃, TMS als interner Standard): δ = 0,80-0,84 ppm (t,3H), 1,03 ppm (s,6H), 1,39 ppm (s,3H), 1,45-1,48 ppm (m,2H), 1,59-1,65 ppm (m,2H), 1,72 ppm (s,3H), 1,74-1,84 ppm (m,2H), 1,97-2,04 ppm (m,17H), 4,14 ppm (s,1H), 6,11-6,47 ppm (m,9H), 6,54-6,71 ppm (m,7H), 7,55+7,58 ppm (d,1H).

### Beispiel 15

### Umsetzung von β-Apo-4'-carotinal mit 4,4-Diäthyl-5-methylen-1,3-dioxolan-2-on zu 3-Aethyl-3-hydroxy-7,11,15,20,24-pentamethyl-26-(2,6,6-trimethylcyclohex-1-enyl)-hexacosa-5,7,9,11,13,15,17,19,21,23,25-undecaen-4-on

In einem mit Rührer, Thermometer, Kühler und Tropftrichter versehenen 200 ml-Sulfierkolben werden unter Argon 10,00 g (0,0202 Mol) β-Apo-4'-carotinal in 30 ml Propanol aufgeschlämmt. Zu der resultierenden Suspension werden 6,53 g (0,0403 Mol) 4,4-Diäthyl-5-methylen-1,3-dioxolan-2-on mit 20 ml Propanol dazugespült und anschliessend innert einer Minute 7,52 ml 50%ige Kaliumhydroxidlösung unter Rühren zugetropft. Dabei steigt die Temperatur auf etwa 47°C an. Die violett-rote Suspension wird dann mittels eines Oelbads auf 80°C erhitzt. Nach einer Reaktionszeit von 5 Stunden werden dem Reaktionsgemisch 70 ml entionisiertes Wasser zugegeben, wonach man das Reaktionsgemisch wieder auf 80°C erhitzt und noch eine Stunde bei dieser Temperatur rührt. Anschliessend wird mittels eines Wasserbads auf 25°C abgekühlt und 30 Minuten bei dieser Temperatur nachgerührt. Dann nutscht man die Suspension über eine mit Papierfilter versehene Glasschlitznutsche, presst den erhaltenen Kristallkuchen fest und wäscht ihn zweimal mit jeweils 50 ml Propanol und dreimal mit jeweils 50 ml entionisiertem Wasser, wobei zwischen den Waschvorgängen abgesogen und der Kristallkuchen festgepresst wird, und trocknet ihn schliesslich etwa 16 Stunden bei 50°C unter vermindertem Druck (Vakuumtrockenschrank).

Auf diese Weise erhält man 11,96 g 3-Aethyl-3-hydroxy-7,11,15,20,24-pentamethyl-26-(2,6,6-trimethyl-cyclohex-1-enyl)-hexacosa-5,7,9,11,13,15,17, 19,21,23,25-undecaen-4-on als braune Kristalle. Die Ausbeute entspricht etwa 99,0% der Theorie.
¹H-NMR (CDCl₃, TMS als interner Standard): δ = 0,77+0,79+0,81 ppm (t,6H), 1,05 ppm (s,6H), 1,45-1,48 ppm (m,2H), 1,58-1,64 ppm (m,2H), 1,72 ppm (s,3H), 1,73-1,78 ppm (m,4H), 1,98-2,02 ppm (m,17H), 4,13 ppm (s,1H), 6,11-6,21 ppm (m,3H), 6,25+6,27 ppm (d,1H), 6,31-6,40 ppm (m,4H), 6,44+6,48 ppm (d,1H), 6,56-6,72 ppm (m,7H), 7,55+7,59 ppm (d,1H).

### Beispiel 16

### Umsetzung von β-Apo-4'-carotinal mit 4-Methyl-4-(4-methyl-3-pentenyl)-5-methylen-1,3-dioxolan-2-on zu 1'-Hydroxy-16'-(3-methylbut-2-enyl)-1',2'-dihydrobeta-χ-carotin-2'-on

In einem mit Rührer, Thermometer, Kühler und Tropftrichter versehenen 200 ml-Sulfierkolben werden unter Argon 19,84 g (0,040 Mol) β-Apo-4'-carotinal in 60 ml Isopropanol aufgeschlämmt. Zu der resultierenden Suspension werden 16,53 g 4-Methyl-4-(4-methyl-3-pentenyl)-5-methylen-1,3-dioxolan-2-on mit 40 ml Isopropanol dazugespült und anschliessend innert 2 Minuten 14,81 ml 50%ige Kaliumhydroxidlösung unter Rühren zugetropft. Dabei steigt die Temperatur auf etwa 46°C an. Die violett-rote Suspension wird dann mittels eines Oelbads auf 70°C erhitzt. Nach einer Reaktionszeit von 4,75 Stunden werden dem Reaktionsgemisch 140 ml Wasser zugegeben, wonach man das Reaktionsgemisch wieder auf 70°C erhitzt und noch eine Stunde bei dieser Temperatur rührt. Anschliessend wird mittels eines Wasserbads auf 25°C abgekühlt und 15 Minuten bei dieser Temperatur nachgerührt. Dann filtriert man die Kristalle ab, presst den erhaltenen Kristallkuchen fest und wäscht ihn zweimal mit jeweils 100 ml Isopropanol und dreimal mit jeweils 100 ml Wasser, wobei zwischen den Waschvorgängen abgesogen und der Kristallkuchen festgepresst wird, und trocknet ihn schliesslich etwa 16 Stunden bei 50°C unter vermindertem Druck (Vakuumtrockenschrank).

Auf diese Weise erhält man 23,38 g 1'-Hydroxy-16'-(3-methylbut-2-enyl)-1',2'-dihydrobeta-χ-carotin-2'-on als braune Kristalle. Die Ausbeute entspricht 92,1% der Theorie, allerdings bedarf das Produkt einer weiteren Reinigung.

Zwecks Reinigung werden 22,0 g des Produkts in 220 ml Methylenchlorid gelöst. Zu der Lösung werden unter Rühren innert anderthalb Stunden 220 ml Methanol zugetropft. Dabei erfolgt die Kristallisation, und die resultierende Suspension wird 30 Minuten bei Raumtemperatur nachgerührt. Man filtriert dann die Kristalle über eine Glassinternutsche ab und wäscht sie zweimal mit jeweils 50 ml Methanol. Schliesslich trocknet man die erhaltenen Kristalle etwa 16 Stunden bei 50°C unter vermindertem Druck (Vakuumtrockenschrank).

Auf diese Weise erhält man 17,04 g (67,1% der Theorie) 1'-Hydroxy-16'-(3-methylbut-2-enyl)-1',2'-dihydrobeta-χ-carotin-2'-on als schwarz-graue Kristalle.
¹H-NMR (CDCl₃, TMS als interner Standard): δ = 1,03 ppm (s,6H), 1,38 ppm (s,3H), 1,45-1,48 ppm (m,2H), 1,54 ppm (s,3H), 1,58-1,65 ppm (m,5H), 1,72 ppm (s,3H), 1,73-1,83 ppm (m,3H), 2,00-2,08 ppm (m,17H), 4,16 ppm (s,1H), 5,03-5,05 ppm (m,1H), 6,11-6,77 ppm (m,17H), 7,54+7,58 ppm (d,1H).

## Patentansprüche

1. Verfahren zur Ueberführung eines gegebenenfalls substituierten konjugierten Polyenaldehyds der allgemeinen Formel
A-CHO I'
oder
OHC-B-CHO Iʺ
worin A oder B den gegebenenfalls substituierten konjugierten Polyen-Monoaldehyd bzw. -Dialdehyd ausser der Formylgruppe bzw. der beiden Formylgruppen darstellt und wobei sich die Formylgruppe(n) in der(den) Endstellung(en) der konjugierten Kette dieses Polyenaldehyds befindet bzw. befinden,
in einen Polyen(di)alkohol der allgemeinen Formel
A-CH=CH-CO-CR¹R²OH II'
bzw.
HOR²R¹C-OC-HC=HC-B-CH=CH-CO-CR¹R²OH IIʺ
worin
R¹ eine C₁₋₆-Alkylgruppe und
R² eine C₁₋₆-Alkylgruppe oder eine C₂₋₆-Alkenylgruppe bedeuten, oder
R¹ und R² zusammen 1,4-Tetramethylen oder 1,5-Pentamethylen bilden,
**dadurch gekennzeichnet, dass** man den Polyenaldehyd unter basischen Bedingungen mit Cyclocarbonat oder einem Derivat davon der allgemeinen Formel 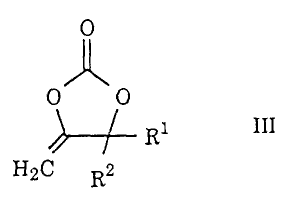 worin R¹ und R² die oben angegebenen Bedeutungen besitzen, umsetzt.

2. Verfahren nach Anspruch 1, **dadurch gekennzeichnet, dass** als Polyenaldehyd eine Verbindung der allgemeinen Formel
R - CHO I
worin
R eine Gruppe (a), (b) oder (c) 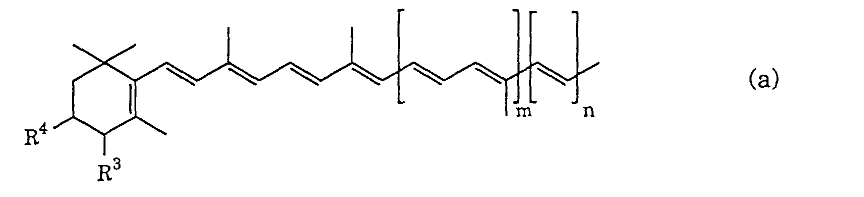 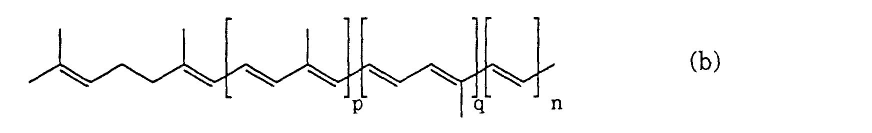 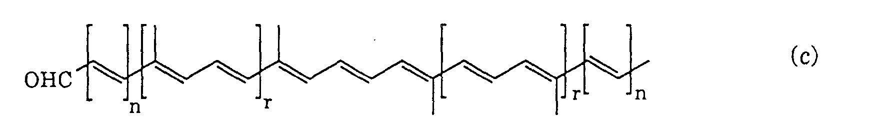 bedeutet, in der
R³ und R⁴ unabhängig voneinander Wasserstoff, eine gegebenenfalls geschützte Hydroxygruppe oder eine gegebenenfalls geschützte Oxogruppe,
m 0, 1, 2, 3 oder 4,
n 0 oder 1,
p 0, 1 oder 2,
q 0, 1, 2 oder 3
und
r 0, 1 oder 2 bedeuten,
wobei im Falle der Verbindung der Formel I, in der R die Gruppe (c) bedeutet, eine der beiden Formylgruppen gegebenenfalls geschützt ist, unter basischen Bedingungen mit Cyclocarbonat oder einem Derivat davon der Formel III zu einer Verbindung der allgemeinen Formel
R'-CH=CH-CO-CR¹R²OH II
worin R' die oben angegebene Bedeutung von R hat, wobei, im Falle, dass R' eine Gruppe (c) bedeutet, die Formylgruppe entweder geschützt oder durch die Gruppe HOR²R¹C-CO-CH=CH- ersetzt ist,
umgesetzt wird.

3. Verfahren nach Anspruch 1 oder 2, **dadurch gekennzeichnet, dass** man den Polyenaldehyd mit 1 bis 5 Aequivalenten des Cyclocarbonats bzw. Cyclocarbonatderivats der Formel III in einem organischen oder wässrigorganischen Lösungsmittel bei Temperaturen im Bereich von 25°C bis 120°C und in Gegenwart einer Base umsetzt.

4. Verfahren nach Anspruch 3, **dadurch gekennzeichnet, dass** das organische Lösungsmittel ein niederer Alkohol mit 1 bis 3 Kohlenstoffatomen, ein cyclischer Aether, ein Aromat oder Methylenchlorid ist, der jeweils gegebenenfalls im Gemisch mit Wasser verwendet wird.

5. Verfahren nach Anspruch 3 oder 4, **dadurch gekennzeichnet, dass** als Base ein Alkalihydroxid oder ein Alkalialkoxid verwendet wird.

6. Verfahren nach einem der Ansprüche 3 bis 5, **dadurch gekennzeichnet, dass** die Umsetzung bei Temperaturen von 40°C bis 80°C erfolgt.

7. Verfahren nach einem der Ansprüche 2 bis 6, **dadurch gekennzeichnet, dass** man von einem so hergestellten Produkt der Formel II, das eine oder mehrere Schutzgruppen aufweist, die vorhandene(n) Schutzgruppe(n) abspaltet.

8. Verfahren nach einem der Ansprüche 1 bis 7, **dadurch gekennzeichnet, dass** als Reagens der Formel III Cyclocarbonat selber verwendet wird.

## Claims

1. A process for the conversion of an optionally substituted conjugated polyene aldehyde of the general formula
A-CHO I'
or
OHC-B-CHO Iʺ
wherein A or B represents the optionally substituted conjugated polyene monoaldehyde or, respectively, dialdehyde devoid of the formyl group or, respectively, of the two formyl groups, with the formyl group(s) being situated in the terminal position of the conjugated chain of this polyene aldehyde,
into a polyene (di)alcohol of the general formula
A-CH=CH-CO-CR¹R²OH II'
or, respectively,
HOR²R¹C-OC-HC=HC-B-CH=CH-CO-CR¹R²OH IIʺ
wherein
R¹ signifies a C₁₋₆-alkyl group and
R² signifies a C₁₋₆-alkyl group or a C₂₋₆-alkenyl group or
R¹ and R² together form 1,4-tetramethylene or 1,5-pentamethylene,
which process comprises reacting the polyene aldehyde under basic conditions with cyclocarbonate or a derivative thereof of the general formula 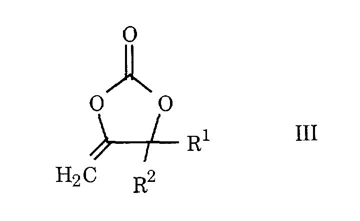 wherein R¹ and R² have the significances given above.

2. A process according to claim 1, wherein a compound of the general formula
R-CHO I
wherein
R signifies a group (a), (b) or (c) 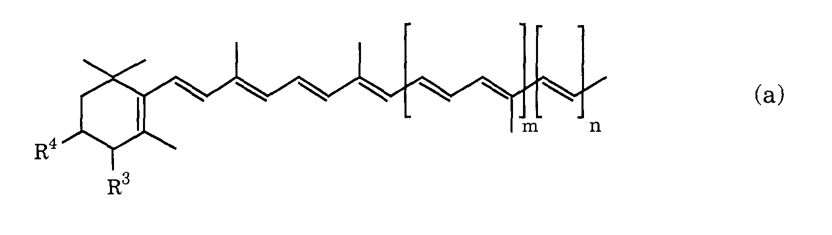 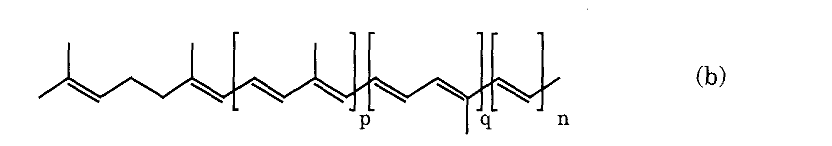 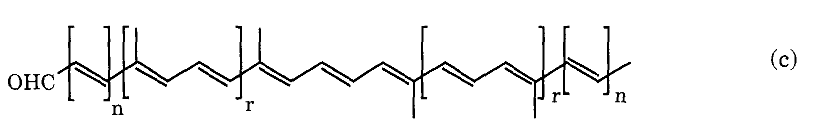 in which
R³ and R⁴ each independently signify hydrogen, an optionally protected hydroxy group or an optionally protected oxo group,
m signifies 0, 1, 2, 3 or 4,
n signifies 0 or 1,
p signifies 0, 1 or 2,
q signifies 0, 1, 2 or 3
and
r signifies 0, 1 or 2,
with one of the two formyl groups being optionally protected in the case of the compound of formula I in which R signifies group (c),
is reacted as the polyene aldehyde under basic conditions with cyclocarbonate or a derivative thereof of formula III to give a compound of the general formula
R'-CH=CH-CO-CR¹R²OH II
wherein R' has the significance of R given above, with the formyl group being either protected or replaced by the HOR²R¹C-CO-CH=CH- group when R' signifies group (c).

3. A process according to claim 1 or 2, wherein the polyene aldehyde is reacted with 1 to 5 equivalents of the cyclocarbonate or cyclocarbonate derivative of formula III in an organic or aqueous-organic solvent at temperatures in the range of 25°C to 120°C and in the presence of a base.

4. A process according to claim 3, wherein the organic solvent is a lower alcohol with 1 to 3 carbon atoms, a cyclic ether, an aromatic or methylene chloride, each of which is optionally used in admixture with water.

5. A process according to claim 3 or 4, wherein an alkali hydroxide or an alkali alkoxide is used as the base.

6. A process according to any one of claims 3 to 5, wherein the reaction is carried out at temperatures from 40°C to 80°C.

7. A process according to any one of claims 2 to 6, wherein the protecting group(s) present is/are cleaved off from a thus-manufactured product of formula II which contains one or more protecting groups.

8. A process according to any one of claims 1 to 7, wherein cyclocarbonate itself is used as the reagent of formula III.

## Revendications

1. Procédé pour la conversion d'un polyène-aldéhyde conjugué, éventuellement substitué, de formule générale
A-CHO I'
ou
OHC-B-CHO Iʺ
où A ou B représente le polyène-monoaldéhyde ou -dialdéhyde conjugué éventuellement substitué hormis le groupe formyle ou les deux groupes formyle, et le(s) groupe(s) formyle se trouvant dans la(les) positions(s) terminale(s) de la chaîne conjuguée de ce polyène-aldéhyde,
en un polyène-(di)alcool de formule générale
A-CH=CH-CO-CR¹R²OH II'
ou
HOR²R¹C-OC-HC=HC-B-CH=CH-CO-CR¹R²OH IIʺ
où
R¹ représente un groupe alkyle en C₁-C₆ et
R² représente un groupe alkyle en C₁-C₆ ou un groupe alcényle en C₂-C₆,
ou
R¹ et R² forment ensemble un groupe 1,4-tétraméthylène ou 1,5-pentaméthylène,
**caractérisé en ce qu'**on fait réagir le polyène-aldéhyde, dans des conditions basiques, avec un carbonate cyclique ou un dérivé de celui-ci, de formule généraie 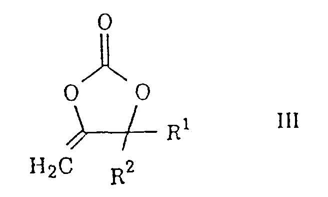 dans laquelle R¹ et R² ont les significations indiquées ci-dessus.

2. Procédé selon la revendication 1, **caractérisé en ce qu'**on fait réagir en tant que polyène-aldéhyde un composé de formule générale
R-CHO I
dans laquelle
R représente un groupe (a), (b) ou (c) 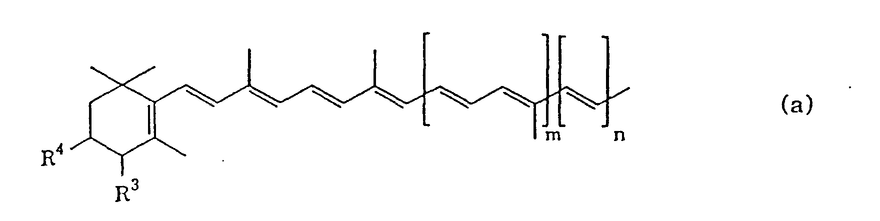 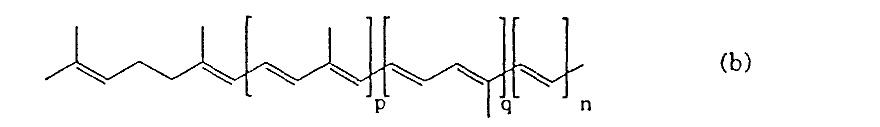 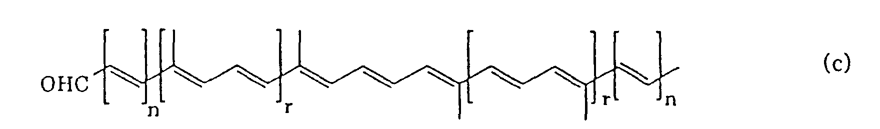 groupes dans lesquels
R³ et R⁴ représentent, indépendamment l'un de l'autre, un atome d'hydrogène, un groupe hydroxy éventuellement protégé ou un groupe oxo éventuellement protégé,
m représente 0, 1, 2, 3 ou 4,
n représente 0 ou 1,
p représente 0, 1 ou 2,
q représente 0, 1, 2 ou 3
et
r représente 0, 1 ou 2,
dans le cas du composé de formule I dans lequel R représente le groupe (c), l'un des deux groupes formyle étant éventuellement protégé,
dans des conditions basiques, avec un carbonate cyclique ou un dérivé de celui-ci, de formule III, pour aboutir à un composé de formule
R'-CH=CH-CO-CR¹R²OH II
dans laquelle R' a la signification de R indiquée plus haut, dans le cas où R' représente un groupe (c), le groupe formyle étant soit remplacé par le groupe HOR²R¹C-CO-CH=CH-, soit protégé.

3. Procédé selon la revendication 1 ou 2, **caractérisé en ce qu'**on fait réagir le polyène-aldéhyde avec 1 à 5 équivalents du carbonate cyclique ou dérivé de carbonate cyclique de formule III dans un solvant organique ou aqueux-organique, à des températures dans la plage de 25°C à 120°C et en présence d'une base.

4. Procédé selon la revendication 3, **caractérisé en ce que** le solvant organique est un alcool inférieur ayant de 1 à 3 atomes de carbone, un éther cyclique, un composé aromatique ou le chlorure de méthylène, qui est dans chaque cas utilisé éventuellement en mélange avec de l'eau.

5. Procédé selon la revendication 3 ou 4, **caractérisé en ce qu'**on utilise comme base un hydroxyde de métal alcalin ou un alcoolate d métal alcalin.

6. Procédé selon l'une quelconque des revendications 3 à 5, **caractérisé en ce qu'**on effectue la réaction à des températures de 40°C à 80°C.

7. Procédé selon l'une quelconque des revendications 2 à 6, **caractérisé en ce qu'**on élimine le(s) groupe(s) protecteur(s) présent(s) d'un produit de formule II ainsi obtenu, qui comporte un ou plusieurs groupes protecteurs.

8. Procédé selon l'une quelconque des revendications 1 à 7, **caractérisé en ce qu'**on utilise comme réactif de formule III le carbonate cyclique lui-même.
